# EUROPEAN PATENT SPECIFICATION

(11) **EP 1 819 392 B1**
(45) Date of publication and mention of the grant of the patent: **20.10.2010**
(21) Application number: 05820710.1
(22) Date of filing: 22.11.2005
(51) Int. Cl.: A61M 31/00, A61B 5/07

(54) **ELECTRONICALLY CONTROLLED PILL**
ELEKTRONISCH GESTEUERTE TABLETTE
PILULE CONTROLEE ELECTRONIQUEMENT

(30) Priority: 29.11.2004 US 631505 P
(43) Date of publication of application: 22.08.2007
(73) Proprietor: Koninklijke Philips Electronics N.V., 5621 BA Eindhoven (NL)
(72) Inventor: LANGEREIS, Gerardus, Rudolph, Briarcliff Manor, New York 10510-8001 (US); LIKOUREZOS, George, Briarcliff Manor, New York 10510-8001 (US)
(74) Representative: Golla-Franz, Anke Lucia
(86) International application number: PCT/IB2005/053863
(87) International publication number: WO 2006/056944

(56) References cited:
- WO-A-2004/066903
- FR-A- 2 591 095
- FR-A- 2 835 730
- GB-A- 2 374 149

## Description

The present disclosure relates generally to medication delivery systems. More particularly, the present disclosure it relates to an electronically controlled pill and system having at least one sensor for delivering at least one medicament.

A medicament, such as aspirin, taken by the person generally traverses the gastrointestinal (GI) tract where it is absorbed for treating an ailment or condition. Objects typically pass through the GI tract in 20-40 hours. Several medicaments are available as time-release capsules for releasing portions of the medicament into the body at different times. Time-release capsules utilize chemical reactions between chemical substances in the gastrointestinal tract and the coating of the capsules for dissolving and releasing the medicament Food, particularly proteins and fats, and the GI chemistry affect the speed of the journey of medicaments through the stomach. As such, medicaments, including medicaments available as time-release capsules, do not follow an exact dispensing or dissolving pattern while traveling through the GI tract.

For example, one person may have more than a "normal" amount of chemical substances in the gastrointestinal tract due to a condition, an earlier-administered medicament, etc. and therefore, cause the coating of the time-release capsule to react quicker than normal. Accordingly, the medicament is released by the time-release capsule at a faster rate than an intended rate. However, another person may have less than the "normal" amount of chemical substance in the gastrointestinal tract and cause the coating of the time-release capsule to react slower than normal, thereby releasing the medicament at a slower rate than the intended rate.

WO 2004/066903 A2 describes an apparatus for drug administration, including an ingestible capsule. The capsule includes a drug, stored by the capsule. An environmentally-sensitive mechanism (e.g., coating) is adapted to change a state thereof responsive to a disposition of the capsule within a gastrointestinal tract of a subject. A driving mechanism, in response to a change of state of the environmentally-sensitive mechanism, is adapted to drive the drug directly through an endothelial layer of the gastrointestinal tract.

GB 2 374 149 A describes a swallowable intra-body drug dispensing capsule which comprises a capsule in which is disposed a sensing module, a bio-active substance dispenser and a component including memory and logic. The sensing module detects one or more biological conditions within a body. This data is evaluated and compared to predetermined criteria stored in the memory in order to trigger drug release (this may be at a variable rate). The memory may include an atomic resolution storage device, and the sensed data may be recorded and stored in the memory. The capsule may also include wireless communication interface for transmitting data and receiving control instructions whilst within the body.

The present disclosure provides an electronically controlled pill or medicament delivery system having at least one sensor for delivering or dispensing a medicament. The dispensing of the medicament is based on location detection using at least one sensor reading, i.e., at least one sensed condition or parameter, such as pH, level of conductivity (water content), etc., taken by the at least one sensor along the gastrointestinal tract. For example, for a normal patient, if the at least one sensor senses a low pH level, the electronically controlled pill can determine that it is located within the stomach. If the pH level begins to rise, the electronically controlled pill can determine that it is exiting the stomach and entering the small intestine.

The electronically controlled pill includes decision and control logic circuitry for controlling the opening and closing of a valve, pump or hatch according to the sensed conditions for dispensing a medicament stored within a medicament reservoir of the pill. Preferably, after the electronically controlled pill is swallowed the one or more sensors are read out continuously and the data is provided to the decision and control logic circuitry. At least one processor of the logic circuitry analyzes the data and determines the relative position of the pill along the gastrointestinal tract. The position of the pill can be determined by accessing one or more look-up tables stored within the processor. The look-up tables preferably correlate the one or more sensed conditions with relative positions along the gastrointestinal tract.

Once the relative position is determined, the decision and control logic circuitry determines whether to control the opening and closing of the valve to dispense the medicament stored within the medicament reservoir. The pill is programmed with the locations or positions it is to dispense the medicament. Therefore, if the determined relative position substantially corresponds with at least one preprogrammed position, the logic circuitry transmits a signal to the valve for opening the valve. The voltage level of the signal determines the amount of opening of the valve.

It is envisioned that the pill is custom programmed or designed according to a patient's medical profile or preexisting ailments which can alter the sensed conditions, such as pH, level of conductivity (water content), etc., along the gastrointestinal tract.

Various embodiments of the present disclosure will be described herein below with reference to the figures wherein:
FIG. 1 is a schematic diagram of an electronically controlled pill having at least one sensor in accordance with the present invention; and
FIG. 2 is a block diagram of the electronically controlled pill having the at least one sensor in accordance with the present invention.

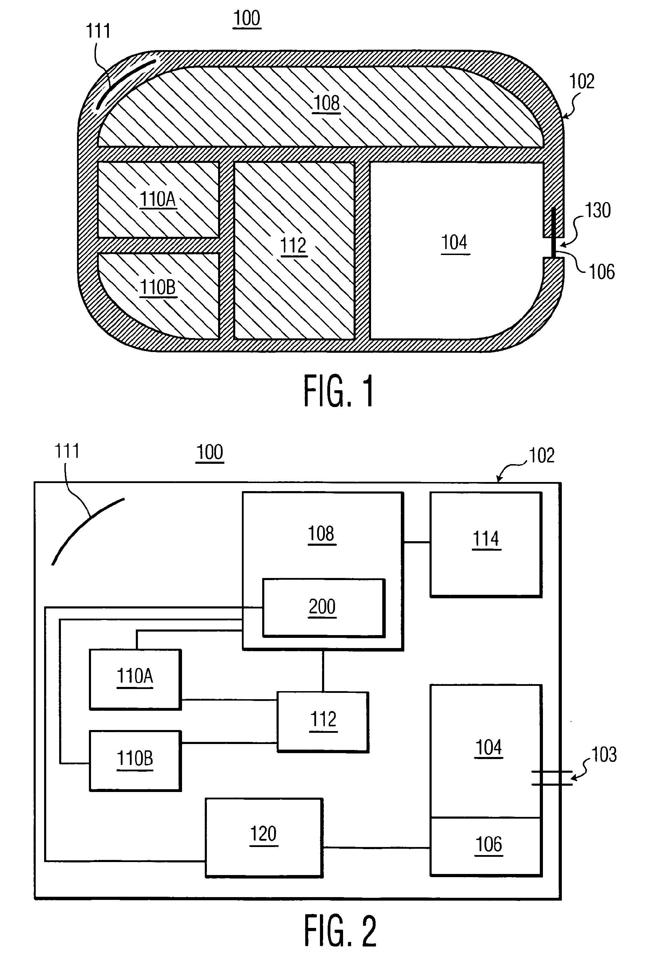

An electronically controlled pill or medicament delivery system according to the present invention is shown by FIGs. 1 and 2, and further described with specificity hereinafter. The electronically controlled pill 100 is a self-contained, electronically controlled medicine delivery system. As described in detail below, the electronically controlled pill 100 includes programmed electronics that control a release mechanism in accordance with at least one sensed condition or parameter, such as pH, level of conductivity (water content), etc., along the gastrointestinal tract for dispensing a medicament. The pill 100 is made from bio-compatibles materials such that the pill 100 is bio-compatible for at least the amount of time it requires to traverse the gastrointestinal tract. The bio-compatible materials are preferably stable in room temperature, such that the pill has a long shelf life.

As used herein and in the claims the word "medicament" refers to medicines, non-medicinal substances, contrast agents, gases, fluids, liquids, chemicals, radiological agents, imaging markers, sensors for monitoring the person's vitals, and other substances capable of being dispensed by the pill 100.

The electronically controlled pill 100 includes an outer shell or housing 102 defining an opening 103; a medicament reservoir 104 for storing a medicament; an electronically controlled release valve, pump or hatch 106 for dispensing the medicaments stored in the medicament reservoir 104 via the opening 103; decision and control logic circuitry 108 for opening and closing the valve 106; and at least one sensor 110 (sensors 110A and 110B are shown in FIGs. 1 and 2). The pill 100 further includes a battery 112 for powering the various components of the pill 100. The decision and control logic circuitry 108 opens and closes the valve 106 in accordance with conditions sensed by the at least one sensor 110 as further described below. The pill 100 further includes a release controller 120 in communication with said circuitry means adapted for interpreting a signal transmitted by the logic circuitry 108 and including microfluidic means for controlling the amount and/or duration of the valve opening in response to said signal from said logic circuitry (108).

Preferably, the shell 102 is resistant to body fluids such as gastric acid and gall from the bile. The shell 102 is preferably manufactured from materials used to fabricate implantable devices, including pacemaker leads and cardiac prosthesis devices, such as artificial hearts, heart valves, intraaortic balloons, and ventricular assist devices. These materials include titanium, Pellethane® 2363 polyetherurethane series of materials available from Dow Chemical Company and Elasthane polyetherurethane available from the Polymer Technology Group, Inc.. Other materials include PurSil® and CarboSil® also available from the Polymer Technology Group, Inc.

At least a portion of the shell 102 preferably includes a metallic liner 111 as shown by FIG. 1 for use in detecting the location of the pill 100 along the gastrointestinal tract by placing a magnetic detector on the patient. When the magnetic detector senses the metallic liner 111, one can easily verify the location of the pill 100 along the gastrointestinal tract. The shell 102 can include one or more other devices or substances, other than the metallic liner 111, such as RF devices, antennas, radioluscent substances, imaging markers, infrared detectors, etc., for enabling detection of the pill (100) from outside the patient.

Preferably, after the electronically controlled pill 100 is swallowed the one or more sensor readings from one or both of the sensors 110A, 110B are read out continuously and the data is provided to the decision and control logic circuitry 108 which includes at least one processor 200. The at least one processor 200 analyzes the data and determines the relative position of the pill 100 along the gastrointestinal tract. The position of the pill 100 can be determined by accessing one or more look-up tables or other data structures stored within the processor 200. The look-up tables correlate the one or more sensor readings or sensed conditions with relative positions along the gastrointestinal tract. An exemplary look-up table correlating sensed pH levels with a respective relative position along the gastrointestinal tract is shown by the following Table.

| pH Level | Position-Gastrointestinal Tract |
|---|---|
| 7.4-7.7 | Mouth |
| 6.3-6.9 | Esophagus |
| 4.0-4.8 | Stomach |
| 7.0-9.0 | Small Intestine |
| 4.0-6.5 | Colon |

Preferably, the at least one processor 200 includes timing circuitry for timing the time the pill 100 is traversing the gastrointestinal tract. Based on a specific time at any given moment, the at least one processor 200 is programmed to determine which data to analyze, i.e., data provided by sensor 110A or data provided by sensor 110B, or both. For example, from two minutes to three minutes after the pill 100 is administered, the at least one processor 200 is programmed to analyze data from sensor 110A. From three minutes to five minutes after the pill 100 is administered, the at least one processor 200 is programmed to analyze data from sensor 110B. From five minutes to ten minutes after the pill 100 is administered, the at least one processor 200 is programmed to analyze data from both sensors 110A, 110B. The time provided by the timing circuitry can also be correlated with a look-up table stored within the at least one processor 200 to determine where along the gastrointestinal tract the pill 100 is at any given time after being administered.

Once the relative position is determined, the decision and control logic circuitry 108 determines whether to control the opening and closing of the valve 106 to dispense the medicament stored within the medicament reservoir 104. The pill 100 is programmed with the locations or positions it is to dispense the medicament. Therefore, if the determined relative position substantially corresponds with at least one preprogrammed position as determined by the logic circuitry 108 using, for example, a comparator, the logic circuitry 108 transmits a signal to the release controller 120 for controlling the valve 106. The release controller 120 is operatively associated or in operative communication with the valve 106 for opening the valve 106. The release controller 120 includes circuitry for interpreting the signal transmitted by the logic circuitry 108 and controlling the amount of the valve opening.

Accordingly, when the pill 100 reaches the target location, the valve 106 opens under the control of the logic circuitry 108 and the release controller 120 and the drug dispenses from the medicament reservoir 104. By opening the valve 106 partially, or by pumping slowly using a pump valve, the medicament dispenses in a controlled manner. Since the logic circuitry 108 controls the dispensing of the medicament, the medicament, in essence, dispenses in accordance with a release profile. An exemplary release profile entails the dispensing of the medicament when the pill 100 is traversing the small intestine.

In accordance with the present invention, a preferred release profile is adhered to during the pill's travel through the gastrointestinal tract, since the decision and control logic circuitry 108 is programmed for closing the valve 106 and controlling the amount the valve 106 is opened for controlling the size of the valve opening. By controlling the size of the valve opening or frequency of valve opening, such as is enabled by microfluidic systems of inkjet printers and the like, the electronically controlled pill 100 can precisely control the quantity of medicament released following one or more sensed conditions by the sensors 110A, 110B.

The voltage level of a signal relayed to the release controller 120 of the valve 106 by the at least one processor 200 determines the size of the valve opening for controlling the quantity of the medicament dispensed at a particular locale along the gastrointestinal tract. When dispensing of the medicament is to be terminated, another signal is transmitted to the release controller 120 of the valve 106 by the at least one processor 200 for closing the valve 106.

The logic circuitry 108 determines to terminate dispensing of the medicament by continuously correlating at least one sensed condition with the relative position of the pill 100 along the gastrointestinal tract using a look-up table. As stated above, the pill 100 is programmed with the locations or positions it is to dispense the medicament. Therefore, if the determined relative position does not substantially correspond with at least one preprogrammed position as determined by the logic circuitry 108 using, for example, the comparator, the logic circuitry 108 transmits a signal to the release controller 120 for closing the valve 106.

The release controller 120 is preferably a micro-electromechanical mechanism capable of receiving the signal from the at least one processor 200 and generating a signal having a variable voltage level to the electronically controlled valve 106 for closing the valve 106 and controlling the size of the valve opening or degree of opening of the valve 106 (in accordance with the voltage level of the received signal). In the simplest case, the release controller 120 is a transistor or D/A circuit that provides voltages to the valve 106 causing it to open or close.

The electronically controlled valve 106 is preferably a micro-electromechanical mechanism, such as a MEMS-valve, capable of being electrically controlled by a signal capable of having a variable voltage levels. Each voltage level corresponds to a different size opening for the valve opening and one voltage level (or no voltage at all, i.e., no signal) corresponds to the valve 106 being closed. The valve 106 is similar in operation to valves used in ink-jet printers for dispensing ink in accordance with the amount that the valve is opened. The valve 106 is characterized as a microfluidic valve for controlling the movement of minute amount of liquids or gases in a miniaturized system.

It is envisioned that the pill 100 is custom programmed or designed according to a patient's medical profile or preexisting ailments which can alter the sensed conditions, such as pH, level of conductivity (water content), etc., along the gastrointestinal tract. With reference to FIG. 2, the decision and control logic circuitry 108 includes a start timer mechanism 114 for causing the activation of the logic circuitry 108 and the sensors 110A, 110B for continuously reading out data. In a preferred embodiment, the start timer mechanism 114 is a micro-electromechanical (MEM) mechanism having a sensor 116 for sensing the presence of a liquid, such as water, saliva, etc. When the pill 100 is taken or administered, the sensor 116 senses the presence of a liquid, and transmits an electrical signal to the logic circuitry 108 for activation thereof. In turn, the logic circuitry 108 transmits a signal to the sensors 110A, 110B for activation thereof and the continuous read out of data.

In an alternate embodiment, the start timer mechanism 114 is a button which is pushed to transmit the electrical signal to the logic circuitry 108. The button is pushed just before the pill 100 is administered to a person or animal.

In another embodiment, activation of the logic circuitry 108 and the sensors 110A, 110B is achieved by dissolving a thin, water soluble coating that separates two electrical contacts of a switch, thereby enabling the switch to close the circuit. In still another embodiment, the switch is manually triggered by the patient or caregiver.

One skilled in the art can appreciate that the electronically controlled pill 100 in accordance with the present disclosure is suitable for dosing pharmaceutical components which are hard to dose using soluble capsules or pressed pills that might harm the mouth or stomach, or that might be damaged themselves in the mouth or stomach. Fluid phase drugs are also easier to dose using the pill 100 of the present disclosure than using conventional pills.

Preferably, the at least one processor 200 stores the data received from the sensors 110A, 110B, such that the data can be retrieved once the pill 100 passes through the gastrointestinal tract. The data can also be transmitted from within the patient to a data recorder situated outside the patient by fitting the pill 100 with communications circuitry having at least one antenna. The data can be used to determine whether the sensed conditions or parameters are normal. For example, one can determine if the pH levels at various parts of the gastrointestinal tract are within a range considered to be normal. If not, treatment can be administered for correcting the pH levels at one or more parts of the gastrointestinal tract as known in the art, or by administering one or more pills 100 for dispensing at least one medicament for increasing or decreasing the pH level at one or more parts of the gastrointestinal tract.

The described embodiments of the present disclosure are intended to be illustrative rather than restrictive, and are not intended to represent every embodiment of the present disclosure. Various modifications and variations can be made without departing from the scope of the disclosure as set forth in the following claims.

## Claims

1. A medicament delivery system (100) for dispensing a medicament while traversing the gastrointestinal tract, said system (100) comprising:
a housing (102);
a reservoir (104) for storing said medicament within said housing (102);
a valve (106) in fluid communication with said reservoir (104);
at least one sensor (110) for sensing at least one parameter; and
circuitry (108) for controlling said valve (106) for opening and closing said valve (106) for dispensing said medicament from said reservoir (104) in accordance with said at least one sensed parameter;
**characterized by** a release controller (120) in communication with said circuitry (108) for interpreting a signal transmitted by said circuitry (108) and including microfluidic means for controlling the amount and/or duration of the valve opening in response to said signal from said circuitry (108).

2. The system (100) according to Claim 1, wherein said housing (102) is manufactured from at least one material selected from the group consisting of titanium, Pellethane® 2363 polyetherurethane series of materials, Elasthane polyetherurethane, PurSil®, and CarboSil®.

3. The system (100) according to Claim 1, wherein said circuitry comprises at least one processor (200) storing at least one data structure correlating said at least one sensed parameter with a position along the gastrointestinal tract.

4. The system (100) according to Claim 3, wherein said at least one data structure is a look-up table.

5. The system (100) according to Claim 1, further comprising a battery (112) for powering said circuitry (108) and said at least one sensor (110).

6. The system (100) according to Claim 1, wherein said circuitry (108) comprises a start timer mechanism (114) for causing the activation of the logic circuitry (108) and the at least one sensor (110).

7. The system (100) according to Claim 6, wherein the start timer mechanism (114) is a micro-electromechanical (MEM) mechanism having a sensor (116) for sensing the presence of a liquid.

8. The system (100) according to Claim 1, wherein said release controller (120) controls a degree of opening of said valve (106) in accordance with a voltage level of said at least one signal.

9. The system (100) according to Claim 1, wherein the system (100) comprises at least one substance or device for enabling detection of the system (100) from outside the gastrointestinal tract.

10. The system (100) according to Claim 1, wherein said circuitry (108) comprises at least one processor (200) programmed with at least one location along the gastrointestinal tract at which said medicament is to be dispensed.

## Patentansprüche

1. Medikamentabgabesystem (100) zur Abgabe eines Medikaments während des Durchquerens des Magen-Darm-Trakts, wobei das genannte System (100) Folgendes umfasst:
ein Gehäuse (102);
ein Reservoir (104) zum Aufbewahren des genannten Medikaments innerhalb des genannten Gehäuses (102);
ein Ventil ((106) in Fluidkommunikation mit dem genannten Reservoir (104);
mindestens einen Sensor (110) zum Erfassen von mindestens einem Parameter; und
eine Schaltung (108) zum Steuern des genannten Ventils (106) zum Öffnen und Schließen des genannten Ventils (106) für die Abgabe des genannten Medikaments aus dem genannten Reservoir (104) in Übereinstimmung mit dem genannten mindestens einen erfassten Parameter;
**gekennzeichnet durch** eine Freigabe-Steuereinheit (120) in Kommunikation mit der genannten Schaltung (108) zum Interpretieren eines **durch** die genannte Schaltung (108) übertragenen Signals und mit Mikrofluid-Mitteln zum Steuern des Ausmaßes und/oder der Dauer der Ventilöffnung in Reaktion auf das genannte Signal von der genannten Schaltung (108).

2. System (100) nach Anspruch 1, wobei das genannte Gehäuse (102) aus mindestens einem Material aus der Gruppe bestehend aus Titan, der Polyetherurethan-Materialreihe Pellethane® 2363 , Elasthan-Polyetherurethan, PurSil® und CarboSil® hergestellt ist.

3. System (100) nach Anspruch 1, wobei die genannte Schaltung mindestens einen Prozessor (200) zum Speichern von mindestens einer Datenstruktur umfasst, die den genannten mindestens einen erfassten Parameter mit einer Position entlang des Magen-Darm-Trakts korreliert.

4. System (100) nach Anspruch 3, wobei die genannte mindestens eine Datenstruktur eine Nachschlagetabelle ist.

5. System (100) nach Anspruch 1, weiterhin mit einer Batterie (112) für die Stromversorgung der genannten Schaltung (108) und des genannten mindestens einen Sensors (110).

6. System (100) nach Anspruch 1, wobei die genannte Schaltung (108) einen Starttimer-Mechanismus (114) zum Veranlassen der Aktivierung der Logikschaltung (108) und des mindestens einen Sensors (110) umfasst.

7. System (100) nach Anspruch 6, wobei der Starttimer-Mechanismus (114) ein mikro-elektromechanischer (MEM) Mechanismus mit einem Sensor (116) zum Erfassen der Anwesenheit einer Flüssigkeit ist.

8. System (100) nach Anspruch 1, wobei die genannte Freigabe-Steuereinheit (120) einen Öffnungsgrad des genannten Ventils (106) in Übereinstimmung mit einem Spannungspegel des genannten mindestens einen Signals steuert.

9. System (100) nach Anspruch 1, wobei das System (100) mindestens eine Substanz oder Vorrichtung zum Ermöglichen der Detektion des Systems (100) von außerhalb des Magen-Darm-Trakts umfasst.

10. System (100) nach Anspruch 1, wobei die genannte Schaltung (108) mindestens einen Prozessor (200) umfasst, der mit mindestens einer Position entlang des Magen-Darm-Trakts programmiert ist, an der das genannte Medikament abzugeben ist.

## Revendications

1. Système d'administration de médicament (100) pour distribuer un médicament lorsqu'il traverse le tractus gastro-intestinal, ledit système (100) comprenant :
un logement (102) ;
un réservoir (104) pour stocker ledit médicament au sein dudit logement (102) ;
une soupape (106) en communication fluidique avec ledit réservoir (104) ;
au moins un capteur (110) pour détecter au moins un paramètre ; et
une circuiterie (108) pour commander ladite soupape (106) pour ouvrir et fermer ladite soupape (106) pour distribuer ledit médicament dudit réservoir (104) en conformité avec ledit au moins un paramètre détecté ;
**caractérisé par** une unité de commande de libération (120) en communication avec ladite circuiterie (108) pour interpréter un signal transmis par ladite circuiterie (108) et incluant des moyens microfluidiques pour commander la quantité et/ou la durée de l'ouverture de soupape en réponse audit signal provenant de ladite circuiterie (108).

2. Système (100) selon la revendication 1, dans lequel ledit logement (102) est fabriqué à partir d'au moins un matériau choisi dans le groupe consistant en le titane, la gamme de matériaux polyétheruréthane Pellethane®2363, le polyétheruréthane Elasthane, PurSil® et CarboSil®.

3. Système (100) selon la revendication 1, dans lequel ladite circuiterie comprend au moins un processeur (200) stockant au moins une structure de données corrélant ledit au moins un paramètre détecté avec une position le long du tractus gastro-intestinal.

4. Système (100) selon la revendication 3, dans lequel ladite au moins une structure de données est une table de consultation.

5. Système (100) selon la revendication 1, comprenant en outre une batterie (112) pour alimenter ladite circuiterie (108) et ledit au moins un capteur (110).

6. Système (100) selon la revendication 1, dans lequel ladite circuiterie (108) comprend un mécanisme de chronomètre de départ (114) pour provoquer l'activation de la circuiterie logique (108) et du au moins un capteur (110).

7. Système (100) selon la revendication 6, dans lequel le mécanisme de chronomètre de départ (114) est un mécanisme microélectromécanique (MEM) comportant un capteur (116) pour détecter la présence d'un liquide.

8. Système (100) selon la revendication 1, dans lequel ladite unité de commande de libération (120) commande un degré d'ouverture de ladite soupape (106) en conformité avec un niveau de tension dudit au moins un signal.

9. Système (100) selon la revendication 1, dans lequel le système (100) comprend au moins une substance ou un dispositif pour permettre la détection du système (100) depuis l'extérieur du tractus gastro-intestinal.

10. Système (100) selon la revendication 1, dans lequel ladite circuiterie (108) comprend au moins un processeur (200) programmé avec au moins un emplacement le long du tractus gastro-intestinal au niveau duquel ledit médicament doit être distribué.
